# EUROPEAN PATENT APPLICATION

(11) **EP 2 574 321 A1**
(43) Date of publication of application: **03.04.2013**
(21) Application number: 11183633.4
(22) Date of filing: 30.09.2011
(51) Int. Cl.: A61F 13/58

(54) **Disposable Diaper For Adults Having A Non-Woven Closure Tape**

(71) Applicant: BENTO bantcilik ve Temizlik Maddeleri Sanayi Ticaret A.S., 34909 Istanbul (TR)
(72) Inventor: Kus, Ercan, 34909 Istanbul (TR)
(74) Representative: Sevinç, Erkan

(57) **Abstract**

The present invention proposes a closure system for effecting closure of an adult diaper around the abdomen of a wearer wherein said closure system is provided as a continuous non-woven closure tape. Said closure tape comprises a plurality of mechanical fastener portions and a plurality of adhesive fastener portions, each fastener and adhesive portion paralelly extending along the length of said continuous strip in the machine direction. Said plurality of parallel strips of mechanical fastener elements are spaced by adhesive regions in the form of a series of consecutively arranged strip pairs and each of said fastener and adhesive strips are 3 mm wide in the cross-direction.

## Description

### Technical Field of the Invention

The invention relates to an all-in-one manufactured easily attachable closure tape for adult diapers. The closure tape of the present invention comprises a mechanical fastener portion along with an adhesive portion for fastening the closure tape to a suitable location of a diaper for effecting closure of the diaper around the waist of the wearer.

### Background of the Invention

The present invention generally relates to diapers, particularly adult incontinence diapers having larger sizes compared to baby diapers.

Another aspect of adult diapers lies in that the problem has resulted in difficulty in fabricating diapers of a sufficient width to accommodate larger individuals as one of the growing problems in today's world is obesity.

There have been a wide variety of products in the market for fastening larger incontinence garments for adults. These have been generally in the form of pressure sensitive adhesive tapes and more recently mechanical fastening systems derived from the Velcro® products comprising a hook and a loop element.

Some closure systems relying solely on the pressure sensitive adhesive coating of a thermoplastic tape have also been introduced in the market. Such tapes transfer one layer of their thermoplastic components onto the surface of a diaper and rely merely on the pressure sensitive adhesive of the transferred thermoplastic layer to hold the diaper properly in place. The transferred adhesive coated thermoplastic layer generally has a relatively small area and provides extremely poor results, where maintaining tightness requires more shear strength of fastening material.

These closure systems are very much restricted on the number of times the diaper is re-opened and closed due to the degradation of the tack of the adhesive, even if the user pays extreme attention to the cleanliness of the conditions. The degradation in the tack of the adhesive becomes obsolete, if the closure tape is handled with dirty or greasy fingers of the user.

Mechanical fastening systems rely on a hook affixed to a closure tape, in turn affixed onto the ear or side of a diaper. The loop material is usually in the form of a frontal tape which is also designated as a landing zone. When hook material is pressed against the loop material, the resulting bond is satisfactory in terms of its shear and peel strength and closure of the diaper around the abdomen of the wearer can be securely achieved.

A mechanical fastener system may comprise a hook strip affixed to the underside of a fastening tab. When the fastening tab is lifted from the side or ear of a diaper in order to close the diaper around a wearer's abdomen, its hook grips onto the nonwoven (or the like) substrate placed on the front surface of the diaper.

Closure tapes to be employed in adult diapers are to be subject to greater pressure as the product itself is in greater size, therefore necessitating further measures to ascertain proper functioning of the closure tape. To that end, a greater area of landing zone incorporating female mechanical elements is the typical solution to overcome problems of the type defined above.

Further, it is to be noted that a landing zone having an enlarged area is one of the most prominent cost items in terms of materials used and manufacturing routine adapted. It is therefore desirable that an adult diaper product comprising no landing zone at all for effecting satisfactory fastening be provided. The present invention as embodied in Claim 1, features an adult diaper having a closure system requiring no landing zone and is therefore fastenable to a side portion of the diaper chassis conveniently.

### Objects of the Invention

An object of the present invention is to provide a multi layered closure tape that eliminates drawbacks of fastening systems known in the prior art.

Another object of the present invention is to provide a reliable closure system for use with adult diapers, which closure system comprises a mechanical fastening strip that enables the user to repeatedly and effectively re-open and close the incontinence garment to which the closure system is attached.

Another object of the present invention is to provide a reliable closure system for use with adult diapers necessitating no prominent cost increase as reliable fastening is ensured without making use of a landing zone with an enlarged area.

Another object of the present invention is to provide a reliable closure system for use with adult diapers, said closure system being fastenable to a side portion of the diaper chassis conveniently.

### Summary of the Invention

The present invention proposes a closure system for effecting closure of a diaper around the abdomen of a wearer, said closure system being provided as a non-woven closure tape having a plurality of parallel strips of mechanical fastener elements spaced by adhesive regions in the form of a series of consecutively arranged strips.

Each of said mechanical fastener and adhesive region strips extends in the machine direction. Said closure tape comprises a non-woven base with an adhesive applied surface to which said plurality mechanical fastener portions are joined in a spaced manner and regions remaining thereinbetween with adhesive surfaces also contribute to enhance the satisfactory functioning of the closure system.

The closure tape of the invention is suitable for fastening with a suitable non-woven surface portion of the diaper chassis and no special landing zone with mechanical fastening elements such as fastening loops are provided. This provides a far greater range of size adjustability in order to obtain a snug fit around the waist and eliminates use of expansive female mechanical fasteners in a substantially enlarged manner in order for maintaining size adjustability function. This alone eliminates a lot of side-problems and substantially enhances ease of use as adjustability in larger size adult diapers, i.e. in extra extra large diapers, does not pose any additional problem.

### Brief Description of Figures

The figure whose brief explanation is herewith provided is solely intended for providing a better understanding of the present invention and is as such not intended to define the scope of protection or the context in which said scope is interpreted in the absence of the description.

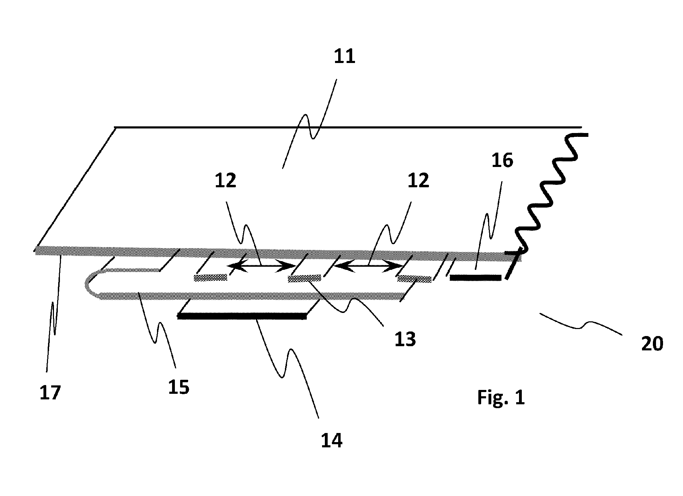

Fig. 1 demonstrates a schematic view of a closure tape for a diaper according to the present invention.

### Detailed Description of the Invention

The present invention provides a closure system with a closure tape having a non-woven base (11) into which a plurality of hook portions (13) are incorporated.

The multi-layered closure system according to the present invention is produced, stored and supplied on a spool which carries a certain length of tape and is designed for continuous feed into a diaper manufacturing line. The required length of tape is cut at right angles to the spool by the diaper manufacturing line and is affixed onto the sides of the diaper. Once the multi-layered closure system of the present invention is affixed to its locations on the diaper, the end user typically lifts the top layers of the closure system. The user affixes the top layer onto a desired location on the front of the diaper pressing firmly on it.

The closure system in the form of a diaper fastening tape according to the present invention provides a construction of a continuous strip generally designated 20 as shown in Fig. 1, which is adapted to be joined to a diaper chassis after being cut in the form of individual fastening tapes (11), each having a plurality of mechanical fastener portions (13).

While said mechanical fastener portions (13) run the length of said continuous strip (20) in the machine direction, a first distal longitudinal edge of said fastening tape (11) is designed as a fingerlift (16) and a second distal longitudinal edge thereof provides diaper chassis connection. Said two distal longitudinal edges define the width of the continuous strip (20) in the cross direction.

Said closure system comprises a non-woven closure tape (11) having a plurality of parallel strips of mechanical fastener elements (13) spaced by adhesive regions (12) in the form of a series of consecutively arranged strips.

Each of said mechanical fastener (13) and adhesive region (12) strips extends in the machine direction. Said closure tape (11) comprises a non-woven base with an adhesive applied surface to which said plurality mechanical fastener portions (13) are joined in a spaced manner and regions remaining thereinbetween with adhesive surfaces (12) also contribute to enhance satisfactory functioning of the closure system.

The closure tape of the invention is suitable for fastening with a suitable non-woven surface portion of the diaper chassis and no special landing zone with mechanical fastening elements such as fastening loops are provided. This provides a far greater range of size adjustability in order to obtain a snug fit around the waist and eliminates use of expansive female mechanical fasteners in a substantially enlarged manner in order for maintaining size adjustability function.

The mechanical fastener portion (13) is adherable to a non-woven closure tape layer (11) and is designed in the form of a hook or microplast® strip preferably around 3-5 mm wide; being affixed to the underside of said closure tape (11). This is typically achieved by means of an adhesive layer (17) applied on the underside of said tape (11).

Various types of hook material can be used to form the hook tabs of the present closure tape. They are generally made of an extruded sheet or strip containing large numbers (around 250 micro hooks per cm²) of mushroom/hexagonal shaped micro hooks on its upper face and is plain on its lower surface.

The micro hooks have extensive gripping properties and grip most surfaces to a certain degree, except thermoplastic derivatives such as polyethylene and polypropylene or co-extruded films. Optimum gripping properties are achieved when the hook is pressed against a loop material that provide in optimum sheer and peel values. These typically range at least 30 N for sheer values (astmd 5170-91) and at least 1.5 N for peel values (astmd 5170-91) when tested on zwick/ roell z 2.5 tester.

The mechanical fastener strips (13) combined with the adhesive strips (12) provides a reliable bond to the diaper's non-woven chassis in the absence of a special landing zone. The width range of said strips starting from at least 3 mm ensures that said bond is maintained during prolonged use.

While the reference 15 denotes the release tape over the fastening and adhesive strips (13, 12), the reference 14 denotes an extra transparent film.

In a nutshell, the present invention proposes a closure system for effecting closure of an adult diaper around the abdomen of a wearer wherein said closure system is provided as a continuous non woven closure tape (11). Said closure tape (11) comprises a plurality of mechanical fastener portions (13) and a plurality of adhesive fastener portions (12), each fastener and adhesive portion (13, 12) paralelly extending along the length of said continuous strip (20) in the machine direction. Said plurality of parallel strips of mechanical fastener elements (13) are spaced by adhesive regions (12) in the form of a series of consecutively arranged strip pairs and each of said fastener and adhesive strips (13, 12) are 3 mm wide in the cross-direction.

## Claims

1. A closure system for effecting closure of an adult diaper around the abdomen of a wearer, said closure system being provided as a continuous non-woven closure tape (11), said tape (11) comprising a plurality of mechanical fastener portions (13) and a plurality of adhesive fastener portions (12), each fastener and adhesive portion (13, 12) paralelly extending along the length of said continuous strip (20) in the machine direction **characterized in that** said plurality of parallel strips of mechanical fastener elements (13) are spaced by adhesive regions (12) in the form of a series of consecutively arranged strip pairs and each of said fastener and adhesive strips (13, 12) are at least 3 mm wide in the cross-direction.

2. A closure system for effecting closure of an adult diaper around the abdomen of a wearer as set forth in Claim 1 wherein there are provided at least three consecutively arranged pairs of mechanical (13) and adhesive strips.

3. A closure system for effecting closure of a diaper around the abdomen of a wearer as set forth in any previous Claims wherein said non-woven tape (11) are silicone coated and UV cured.
